# EUROPÄISCHE PATENTANMELDUNG

(11) **EP 1 369 095 A2**
(43) Veröffentlichungstag der Anmeldung: **10.12.2003**
(21) Anmeldenummer: 02012509.2
(22) Anmeldetag: 04.06.2002
(51) Int. Cl.: A61F 2/00

(54) **Verfahren und Vorrichtung zum Befeuchten eines medizinischen Implantates oder Transplantates**

(71) Anmelder: MTF MediTech Franken GmbH, 90542 Eckental (DE)
(72) Erfinder: Iwatschenko, Peter, 91077 Neunkirchen (DE)
(74) Vertreter: von Hellfeld, Axel, Dr. Dipl.-Phys.

(57) **Zusammenfassung**

Ein Verfahren und eine Vorrichtung zum Befeuchten eines porösen medizinischen Materials sehen vor, dass das Material vor dem Befeuchten evakuiert wird. Die Erfindung beinhaltet auch ein Verfahren zum Herstellen eines porösen medizinischen Materials aus Knochenpartikeln, die zu einem stabilen porösen Körper verpresst werden. Dieser poröse Körper eignet sich besonders für eine Befeuchtung gemäß dem erfindungsgemäßen Verfahren.

## Beschreibung

Die Erfindung betrifft ein Verfahren und eine Vorrichtung zum Befeuchten eines flüssigkeitsaufnehmenden medizinischen Implantates oder Transplantates aus im Wesentlichen biologischem Material.

Ein Implantat ist ein dem (menschlichen) Körper eingesetztes Gewebestück oder sonstiges Material, das zumindest zeitweise im Empfängerkörper verbleibt.

Ein Anwendungsbereich der vorliegenden Erfindung sind insbesondere Transplantate, also Teile eines Gewebes oder eines Organs, die von einem anderen Lebewesen in einen Patienten transplantiert werden. Dabei kommen insbesondere Knochentransplantate in Betracht (human oder bovin). Auch ist es im Stand der Technik bekannt, Partikel aus z.B. Knochen als medizinisches Füllmaterial zu verwenden, z.B. im Dentalbereich zum Füllen von Extraktionshöhlen. Auch derartige Füllmaterialien werden regelmäßig vor dem Einsatz rehydratisiert (also befeuchtet) und hierfür ist die vorliegende Erfindung besonders geeignet. Wenn es gemäß der vorliegenden Erfindung heißt, dass das Material "im Wesentlichen biologisch" sein soll, bedeutet dies, dass das Material zumindest großteils von einem anderen Lebewesen oder auch dem Patienten selbst gewonnen worden ist. Gleichwohl kann das Material gegebenenfalls geringfügige Zusätze (bis zu einigen Prozent, in der Regel weniger als 10 %) Zusätze von nicht-biologischem, d.h. nicht auf die vorstehend genannte Weise gewonnenem Material enthalten.

Nachfolgend wird die Erfindung insbesondere mit Blick auf eine Anwendung zur Befeuchtung von Knochentransplantaten erläutert. Aus dieser Erläuterung ergibt sich dann ohne weiteres auch die Anwendung der Erfindung auf andere poröse medizinische Materialien, wie allgemein biologische und organische Substanzen.

Im Stand der Technik erhält der Chirurg zur Transplantation vorgesehene Knochenstücke in einer sterilen Verpackung. Diese Knochenstücke werden in Kontakt mit einem Befeuchtungsmittel gebracht, z.B. einer Kochsalzlösung oder Plasma. Das Befeuchtungsmittel kann gegebenenfalls auch als Zusatz ein Medikament enthalten. Dieses Verfahren zum Befeuchten von Implantaten bzw. Transplantaten ist aufwendig und kostet Zeit. Es verlangt auch in aller Regel zuviel Befeuchtungsmittel, d.h. es entsteht ein nicht genutzter Überschuss an Befeuchtungsmittel.

Der Erfindung liegt die Aufgabe zugrunde, ein Verfahren und eine Vorrichtung zum Befeuchten poröser medizinischer Substanzen bereitzustellen, mit denen eine einfache Handhabung und ein effektiver Einsatz des Befeuchtungsmittels ermöglicht sind.

Das erfindungsgemäße Verfahren und die erfindungsgemäße Vorrichtung sehen vor, dass die Befeuchtung der porösen medizinischen Substanz durch einen Vakuum-Saugeffekt gefördert wird.

Die Durchdringung der Substanz, also des Implantates bzw. Transplantates mit dem Befeuchtungsmittel wird erfindungsgemäß durch einen Saugeffekt gefördert, der ein Druckgefälle erzeugt, welches das Befeuchtungsmittel in das Implantat bzw. Transplantat treibt. Neben diesem Saugeffekt kann die Befeuchtung des Implantates bzw. Transplantates auch durch andere Effekte wie den Kapillareffekt gefördert werden. Dieser Kapillareffekt ist regelmäßig sowieso vorhanden. Die erfindungsgemäße Saugwirkung hat dem gegenüber aber einen beträchtlichen Vorteil hinsichtlich der Zeit, die für die Befeuchtung erforderlich ist, und auch hinsichtlich der Homogenität der Befeuchtung, d. h. der völlig gleichmäßigen Durchdringung des Materials (des Implantates bzw. des Transplantates) mit dem Befeuchtungsmittel. Auch ermöglicht die Erfindung eine genaue Dosierung der Menge des Befeuchtungsmittels, so dass der Überschuss an Befeuchtungsmittel (also die Menge des Befeuchtungsmittels, die für die Befeuchtung des Materials nicht erforderlich ist) weitgehend reduziert werden kann.

Gemäß der bevorzugten Ausgestaltung der Erfindung ist vorgesehen, das Implantat bzw. Transplantat in zeitlichem Abstand vor dem Befeuchten zu evakuieren. Dabei wird also der Saugeffekt direkt durch das Vakuum in den Poren des Materials erzeugt.

In Abwandlung dieses Ausführungsbeispiels kann der Saugeffekt aber auch durch Evakuierung eines Hohlraumes erzeugt werden, der mit dem Implantat bzw. Transplantat in gasleitende Verbindung bringbar ist. Wird dann kurz vor oder bei dem Befeuchten diese gasleitende Verbindung hergestellt und das Befeuchtungsmittel an einer anderen Stelle dem Implantat bzw. Transplantat zugeführt (z.B. auf der Seite, die der gasleitenden Verbindung gegenüberliegt), wird das Befeuchtungsmittel aufgrund des Saugeffektes quasi in und durch das Material des Implantates bzw. Transplantates gezogen und so die gleichförmige Befeuchtung im Inneren des Materials erreicht. Es erfolgt bei diesem Ausführungsbeispiel die Evakuierung des Materials unmittelbar vor seiner Befeuchtung.

Es ist auch möglich, die Vorrichtung in der Art eines evakuierbaren Behälters zu gestalten mit Wandungen, die sich aufgrund eines Vakuums im Wesentlichen nicht verformen. Es kann dann das zu befeuchtende poröse medizinische Material in diesem Behälter auf Vorrat gehalten werden, wobei das Material des Implantates bzw. des Transplantates ebenfalls evakuiert ist.

Gemäß einer besonders bevorzugten Ausgestaltung sind erfindungsgemäße Vorrichtungen in der Art einer elastischen Vakuumverpackung ausgeführt.

Gemäß einer weiteren bevorzugten Ausgestaltung der Vorrichtungen ist eine erste Kammer vorgesehen, in der das Implantat oder Transplantat enthalten ist, und eine zweite Kammer, in der ein Befeuchtungsmittel, also eine Flüssigkeit, enthalten ist.

Sind dann diese Kammern flüssigkeitsleitend miteinander wahlweise verbindbar, so bewirkt bei Herstellung dieser Verbindung zum Zwecke der Befeuchtung ein Vakuum in der ersten Kammer, dass das Befeuchtungsmittel in das Material des Implantates bzw. Transplantates gesaugt wird.

Eine andere bevorzugte Ausgestaltung der Erfindung sieht vor, dass die Vorrichtung ein Septum aufweist zum Einspritzen einer Flüssigkeit, mit der das Implantat oder Transplantat zumindest teilweise zu befeuchten ist.

Gemäß einer weiteren bevorzugten Ausgestaltung der Erfindung ist vorgesehen, dass das Befeuchtungsmittel ein Medikament enthält.

Es ist auch möglich, die beiden vorstehend erläuterten Konzepte "Zwei-Kammer-System" und "Septum" miteinander derart zu kombinieren, dass die Vorrichtung ein Zwei-Kammer-System mit einer ersten Kammer, in der das evakuierte poröse Material enthalten ist, und einer zweiten Kammer für das Befeuchtungsmittel aufweist, wobei zusätzlich noch an geeigneter Stelle ein Septum vorgesehen sein kann, durch welches zusätzlich zum Befeuchtungsmittel wahlweise ein Medikament einspritzbar ist, so dass es beim Befeuchten zusammen mit dem Befeuchtungsmittel homogen in das Material eindringt. Z.B. kann das Septum so angeordnet werden, dass es ein Einspritzen des Medikamentes in das Befeuchtungsmittel oder in das bereits strömende Befeuchtungsmittel ermöglicht.

Bei dem oben beschriebenen Zwei-Kammer-System mit einer flüssigkeitsleitenden Verbindung dazwischen kann z.B. vorgesehen sein, dass zunächst diese flüssigkeitsleitende Verbindung unterbrochen ist und diese Unterbrechung, z.B. in Form einer Membran oder dergleichen, durch Druck von außen aufbrechbar ist. Ist z.B. die Vorrichtung als elastische Vakuumverpackung ausgeführt, kann der Benutzer durch Druck auf die Kammer, in der das Befeuchtungsmittel enthalten ist, die Membran zum Brechen bringen, so dass das Befeuchtungsmittel in Richtung auf das zu befeuchtende Implantat gesaugt wird.

Gemäß einer weiteren bevorzugten Ausgestaltung, die insbesondere bei Einsatz einer Vakuumverpackung verwendet wird, ist vorgesehen, auf der stromab des Implantates bzw. Transplantates gelegenen Seite einen Freiraum, z.B. in Form einer relativ (im Vergleich zur Elastizität der Folie der Vakuumverpackung) starren Hülse vorzusehen, so dass überschüssige Flüssigkeit, die nach Sättigung des porösen Materials übrig bleibt, vom porösen Material weggesaugt wird, so dass das Material mit genau der erforderlichen Flüssigkeitsmenge beschickt wird und keine Reste an Flüssigkeit, die möglicherweise stören könnten, daran verbleiben.

Es ist auch möglich, dass mit dieser Erfindung insbesondere zu befeuchtende biologische oder organische Material in einem offenen Hohlkörper, wie z.B. einer Hülse anzuordnen. Diese Anordnung in einem offenen Hohlkörper wird insbesondere dann bevorzugt, wenn das Material selbst bei Evakuierung unter Wirkung des äußeren Luftdruckes (also des atmosphärischen Druckes) keine hinreichende Formstabilität aufweist. Aber auch bei gegenüber dem äußeren Luftdruck formstabilem Material kann die Anordnung in einem Hohlkörper vorteilhaft sein. Z.B. kann dadurch der Eintritt des Befeuchtungsmittels in das Material an genau definierter Stelle erfolgen und auch gewährleistet werden, dass das Befeuchtungsmittel vollständig durch das zu befeuchtende biologische oder organische Material durchtritt. Auch überschüssiges Befeuchtungsmittel kann mittels eines solchen Hohlkörpers in einfacher Weise abgeführt werden.

Als Befeuchtungsmittel kommen insbesondere in Betracht eine Kochsalzlösung oder ein Plasma.

Als medizinisches poröses Material kommen insbesondere in Betracht Knochen (Spongiosa) oder knochenähnliche Materialien, z.B. aus Knochen hergestellte Materialien. Die Transplantate können Xenotransplantate oder Allotransplantate sein.

Im Stand der Technik sind insoweit sog. Spongiosa Partikel bekannt, die als sog. Xenotransplantat Verwendung finden (Fa. Tutogen Medical GmbH, D-91077 Neunkirchen am Brand, Deutschland). Bei diesem Stand der Technik werden dem Arzt die Allo- und Xenotransplantate als Partikel bestimmter Größe angeboten, in konservierter, strahlensterilisierter Form, üblicherweise als Granulat.

Derartiges Spongiosa-Granulat wird in einer Vielzahl von chirurgischen Anwendungsbereichen eingesetzt, z.B. in der Orthopädie, der Neurochirurgie, im HNO-Bereich, oder auch in der Dentalmedizin. Die dentalen Anwendungsgebiete umfassen die Augmentation um z.B. Implantaten in Extraktionsalveolen, die Augmentation von Fenestrationsdefekten, weiterhin die lokalisierte Kamm-Augmentation, insbesondere zum Zwecke einer späteren Implantation, die Kammrekonstruktion bei prothetischer Versorgung, die Rekonstruktion von Defekten, die Auffüllung von Extraktionsalveolen, das Füllen von Defekten nach z.B. einer Wurzelspitzenresektion, das Füllen nach einer Zystektomie oder auch das Füllen nach Entfernung retinierter Zähne etc. Auch werden derartige Granulate nach einer Osteotomie eingesetzt. Andere Anwendungsgebiete sind z.B. das Füllen von Craniumdefekten oder Anwendungen im Kieferbereich.

Der Chirurg muss dabei die zuvor üblicherweise präparierten und dehydratisierten Granulate befeuchten. Diese "Rehydratisierung" erfolgt insbesondere mit physiologischer Kochsalzlösung oder mit blutplättchenreichem Plasma (PRP), um das Einheilen zu verbessern. Gegebenenfalls wird auch autogener Knochen untergemischt.

Der Einsatz derartiger Granulate im Stand der Technik erfordert vom Chirurgen nicht nur erhebliche Erfahrung und Geschick beim Rehydratisieren, sondern auch beträchtliches Geschick beim Implantieren in den oben genannten Anwendungsgebieten. Die Forderung nach Erfahrung und Geschick beim Chirurgen bedeutet andererseits die Gefahr von Applikationsfehlern.

Als poröse Körper, zu deren Befeuchtung sich die Erfindung ebenfalls gut eignet, kommen weiterhin in Betracht gepresste Tabletten. Z.B. können solche Tabletten aus den oben beschriebenen Knochenpartikeln (sog. Chips) so verpresst werden, dass die entstehende Tablette porös ist. Allgemein lässt sich dieses Verfahren mit kollagenhaltigen Materialien durchführen.

Die Erfindung betrifft auch ein Verfahren zum Herstellen der vorstehend erläuterten porösen medizinischen Implantate oder Transplantate, bei dem Knochenmaterial zu Partikeln zerkleinert wird und anschließend die Partikel zu porösen Körpern kompaktiert werden. "Kompaktieren" bedeutet in diesem Zusammenhang, dass die Knochenpartikel soweit verdichtet und zusammengepresst werden, dass sie einen formstabilen Verbund bilden, der durchgehend porös ist, ohne wesentliche Beschädigung der Mikroknochenstruktur.

Das Herstellungsverfahren für derartige kompaktierte poröse Tabletten aus Knochen-Partikeln verläuft über ein Malen des Ausgangsknochenmaterials, z.B. in einem Mörser, das Sieben auf geeignete Partikelgrößen, und das anschließende Verpressen zu einer stabilen Tablette. Es hat sich (überraschend) gezeigt, dass allein schon durch ein Verpressen eine relativ formstabile poröse Tablette erhalten werden kann. Wird diese Tablette dann anschließend rehydratisiert, z.B. bevorzugt mit dem oben beschriebenen Vakuum-Saugeffekt, quillt sie etwas auf und wird dadurch sehr gut modellierbar, so dass der Chirurg die Tablette oder Teile davon (es können auch Tabletten unterschiedlicher Größen zur Verfügung gestellt werden) gegebenenfalls direkt am Einsatzort einpassen und modellieren kann.

Die Erfindung schafft hier in doppelter Hinsicht Abhilfe. Zum einen erleichtert der erfindungsgemäß kompaktierte poröse Körper aus Spongiosa-Partikeln als solches schon die Applikation bei einer Vielzahl von Anwendungsbereichen, z.B. den oben genannten, und zum ändern lässt sich der so geformte poröse Körper wesentlich einfacher und besser als das Granulat rehydratisieren, wobei eine Rehydratisierung gemäß der oben beschriebenen Evakuierungstechnik besonders bevorzugt ist. Der Begriff "rehydratisieren" ist hier - dem allgemeinen medizinischen Sprachgebrauch folgend - im weiteren Sinne zu verstehen und umfasst allgemein das Befeuchten mit einer geeigneten Flüssigkeit, nicht nur mit Wasser oder einer Kochsalzlösung. "Rehydratisieren" bedeutet hier also insbesondere auch "ein Befeuchten mit (Blut)-Plasma".

Nachfolgend werden Ausführungsbeispiele der Erfindung anhand der Zeichnung näher erläutert. Es zeigt:
- Figur 1: eine Vorrichtung zum Befeuchten eines porösen medizinischen Materials in auseinandergenommenem Zustand,
- Figur 2A, 2B, 2C und 2D: eine Vorrichtung gemäß Figur 1 in verschiedenen Stadien des Zusammenbaus, und
- Figur 3: ein weiteres Ausführungsbeispiel einer Vorrichtung zum Befeuchten eines saugfähigen medizinischen Materials mit einer Vakuumverpackung.

Die in den Figuren gezeigte Vorrichtung eignet sich besonders zum Befeuchten eines zuvor in bekannter Weise hergestellten und rehydratisierten porösen Knochentransplantates 10. Ein solches Transplantat kann z.B. Abmessungen von typischerweise einigen Zentimetern aufweisen. Das poröse Knochentransplantat 10 kann z.B. ohne Zerkleinerung (Chip-Erzeugung) nur durch Zersägen von Knochen gewonnen werden oder auch gemäß der oben erläuterten Kompaktierung von Partikeln bzw. Chips. Allgemein ausgedrückt steht das bei diesem Ausführungsbeispiel herangezogene Knochentransplantat 10 für einen porösen medizinischen Körper biologischen Ursprungs.

Die Vorrichtung weist ein Unterteil 12 auf aus z.B. durchsichtigem Kunststoff. Beim dargestellten Ausführungsbeispiel ist das Unterteil 12 aus einem sog. Blister-Material, das trotz einer gewissen Elastizität eine hinreichende Festigkeit aufweist, um, je nach Größe des Transplantates 10, bei Anlegen eines Unterdruckes im Inneren auf einigen Quadratzentimetern Fläche gegenüber dem äußeren Luftdruck Formstabilität beizubehalten.

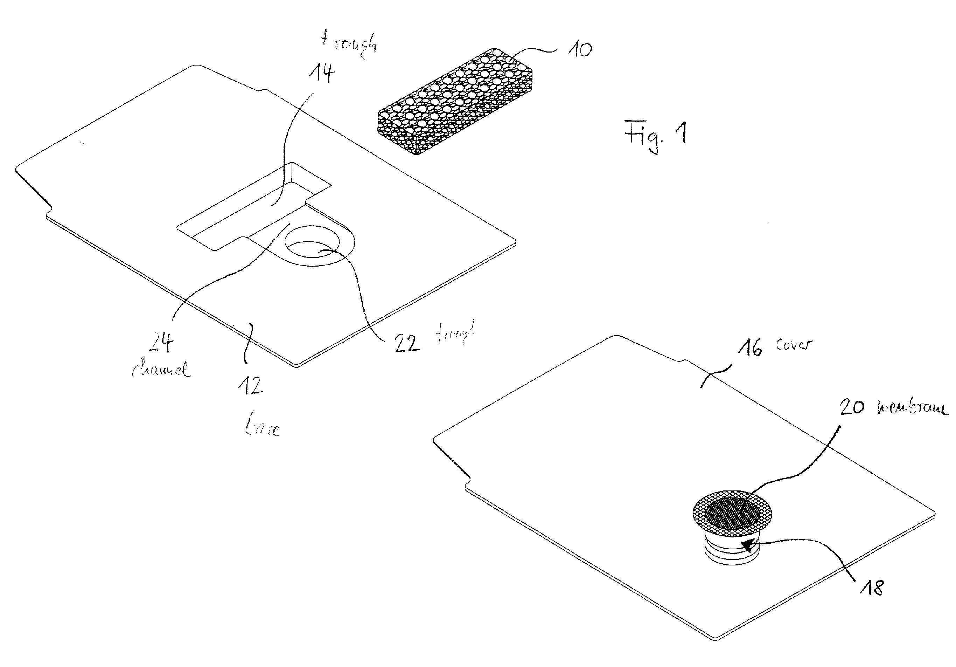

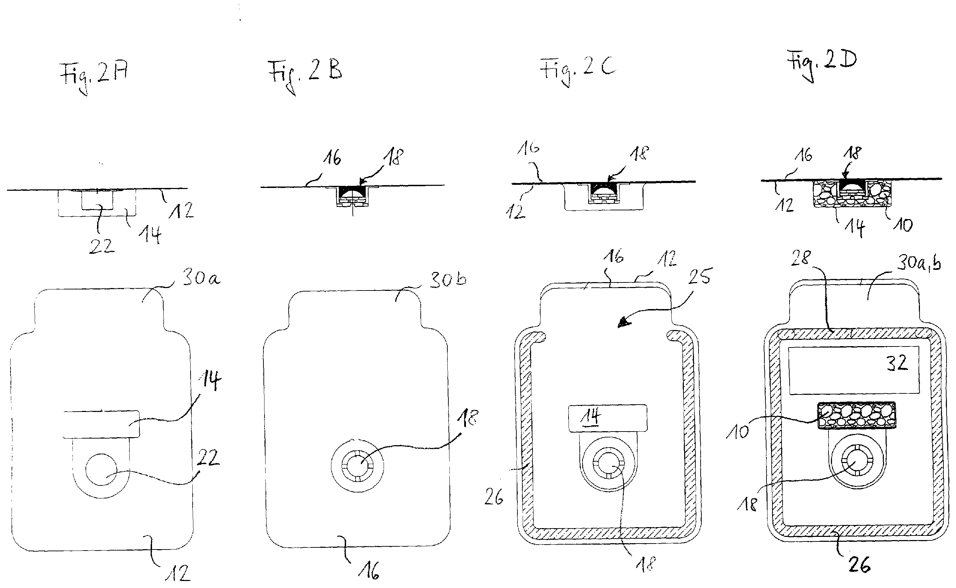

Im Unterteil 12 ist eine Mulde 14 ausgeformt, in die das Transplantat 10genau passt. Ein Oberteil 16 schließt in der Art eines Deckels die Mulde 14 luftdicht gegen die äußere Atmosphäre ab. Ein als solches bekanntes Septum 18 (vgl. auch Figur 2B) ist ebenfalls luftdicht am Oberteil 16 befestigt und ragt in Figur 1 nach unten in eine zweite Mulde 22 im Unterteil 12. Das Septum 18 kann in einer als solches bekannten Bauweise ausgeführt sein und weist oben eine Membran 20 auf, durch die eine Injektionsnadel (nicht gezeigt) durchstechbar ist. Die Begriffe "oben" und "unten" werden hier im Sinne der üblichen Gebrauchsstellung der Vorrichtung verwendet.

Die genannten Mulden 14 und 22 bilden bei auf das Unterteil 12 luftdicht aufgesetztem Oberteil 16 (siehe weiter unten) ein System aus zwei Kammern, die durch einen Kanal 24 miteinander verbunden sind. Beim dargestellten Ausführungsbeispiel ist der Kanal 24 in Form einer relativ flachen und breiten Vertiefung im Unterteil 12 ausgebildet.

Die Figuren 2A bis 2D zeigen einzelne Stufen des Zusammenbaus einer Vorrichtung gemäß Figur 1. Die Figuren 2A bis 2D zeigen jeweils unten in Draufsicht das Unterteil bzw. das Oberteil und darüber einen Querschnitt senkrecht zur Hauptebene des Unterteils in Höhe der Mulde 22, wobei zusätzlich noch die Mulde 14 (die eigentlichen in dem Schnitt nicht enthalten wäre) dargestellt ist.

Zunächst zeigt Figur 2A das Unterteil 12 (Blister) aus einer transparenten Folie mit einer Stärke von 0,2 bis 0,4 mm. Die Größen der Mulden 14 und 22 sind an die Erfordernisse, d.h. die Abmessungen des Transplantates 10 bzw. des vorgesehenen Septums 18 anpassbar.

Figur 2B zeigt das Oberteil 16 mit dem luftdicht daran befestigten Septum 18. Das Oberteil 16 kann z.B. aus einer im Vergleich zum Unterteil elastischeren Deckelfolie mit Stärken im Bereich von 0,1 bis 0,2 mm bestehen.

Figur 2C zeigt das Unterteil 12 und das Oberteil 16 in teilweise zusammengebautem Zustand. Ein Verschweißungsstreifen 26 verbindet Unterteil und Oberteil teilweise, wobei ein Bereich 25 zunächst unverschweißt bleibt, so dass mit einer Vakuumpumpe (nicht gezeigt) Luft aus den Mulden 14, 22 durch den offenen Bereich 25 abgesaugt werden kann. Dabei legen sich Unterteil und Oberteil großflächig aneinander und ein in der Mulde 14 angeordnetes Transplantat 10 wird ebenfalls evakuiert. Durch den oben beschriebenen Kanal 24 zwischen den Mulden 14 und 22 wird auch die dem Septum 18 zugeordnete Mulde 22 sowie auch das Septum evakuiert.

Unterteil 12 und Oberteil 16 sind hinreichend stabil, um die Evakuierung zu ermöglichen (ein abgewandeltes Ausführungsbeispiel mit stärker elastischem Material in der Art einer Vakuumverpackung ist weiter unten erläutert).

Nach der Evakuierung im Zustand gemäß Figur 2C wird ein weiterer Schweißstreifen 28 z.B. thermisch aktiviert oder aufgetragen, der den gesamten Zwischenraum zwischen Unterteil 12 und Oberteil 16 mit Ausnahme zweier Aufreißränder 30a, 30b luftdicht abschließt, zusammen mit dem bereits genannten Klebestreifen 26.

Ein Etikett 32 wird auf dem Oberteil 26 angebracht, um dem Benutzer die erforderlichen Informationen über das Transplantat 10 zu geben. Zum Befeuchten des Transplantates 10 injiziert der Benutzer durch die Membran 20 des Septums 10 eine geeignete Flüssigkeit, z.B. eine Kochsalzlösung oder ein Plasma, gegebenenfalls mit zugesetzten Medikamenten. Die Größe der Mulde 22 ist so an die Größe des Septums angepasst, dass die Flüssigkeit direkt über den Kanal 24 in die Mulde 14 und damit in das Transplantat 10 gelangt. Da die Mulde 22 in der Größe an die äußeren Abmessungen des Septums 18 angepasst ist und ebenso auch die Größe der Mulde 14 an die Größe des Transplantates 10, kann der Benutzer die zugeführte Flüssigkeitsmenge genau an den eigentlichen Bedarf anpassen, also nur soviel Flüssigkeit zugeben, wie zur Befeuchtung des Transplantates 10 erforderlich ist. Z.B. kann die zuzuführende Flüssigkeitsmenge den Benutzer auf dem Etikett 32 angezeigt werden.

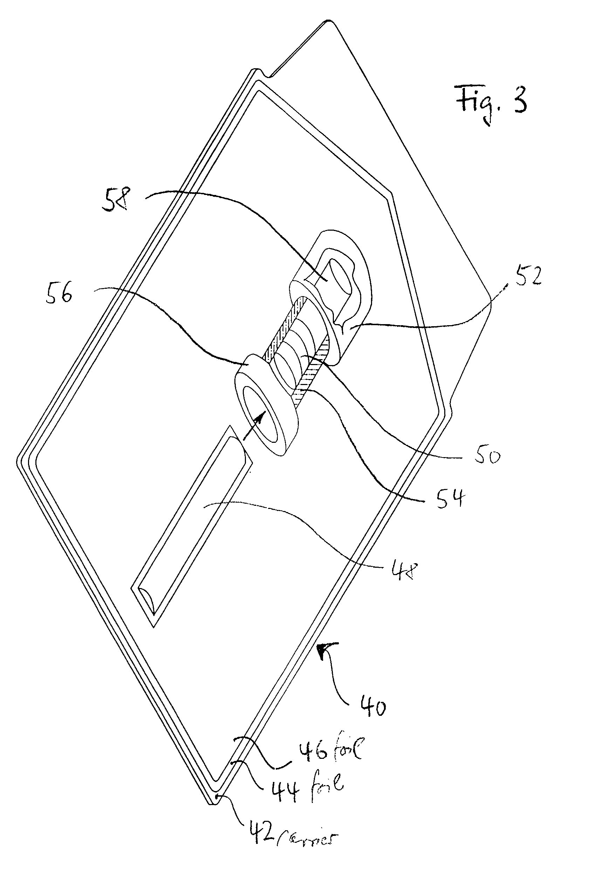

Das anhand der Figuren 1 und 2 dargestellte Ausführungsbeispiel kann z.B. dahingehend abgewandelt werden, dass als Material für die Umhüllung der genannten Teile eine Vakuumverpackung mit voller Elastizität vorgesehen ist. Ein solches Ausführungsbeispiel mit einer Vakuumverpackung ist in Figur 3 schematisch dargestellt. Die Vakuumverpackung 40 weist einen flachen Träger 42 auf, auf den zwei Folien 44, 46 übereinander lamelliert sind. Zwischen den Folien 44, 46 ist ein Flüssigkeitsbehälter 48 wie ein Kissen ausgebildet, das prall mit einer Flüssigkeit gefüllt ist, mit welcher ein medizinisches, flüssigkeitsaufnehmendes Material 50 befeuchtet werden soll. Das Material 50 befindet sich in einer Hülse 45, die hinreichend fest ist, um das Material 50 weitgehend gegen äußere Einflüsse wie extremen Druck oder dergleichen zu schützen. Bevorzugt ist das Material der Hülse 54 bei der vorstehend genannten Formstabilität gleichwohl etwas elastisch. Ein Ring 56 am Eingang zur Hülse 54 sichert die Hülse gegen ein Zuquetschen und ebenso ist am anderen Ende der Hülse 54 ein relativ starrer Aufnahmebehälter 52 für überschüssige Flüssigkeit vorgesehen, in den ein Stutzen 58 ragt, durch den die überschüssige Flüssigkeit in den Behälter 52 eintritt. Das Innere zwischen den Folien 44, 46 einschließlich der Hülse 45 und des Behälters 52 ist evakuiert.

Zur Befeuchtung des flüssigkeitsaufnehmenden Materials 50, also z.B. eines porösen Körpers der obengenannten Art, drückt der Benutzer den Flüssigkeitsbehälter 48 (z.B. mit den Fingern) so stark, dass eine Sollbruchstelle zur Hülse 54 aufbricht, die sich etwa an der Stelle des Pfeiles in Figur 3 zwischen den Folien 44, 46 befindet, so dass Flüssigkeit aus dem Behälter 48 durch den Ring 56 in das innere der Hülse 54 eintritt und dort die Rehydratisierung im oben beschriebenen Sinne durchgeführt wird. Ist das innere des Behälters 52 ebenfalls evakuiert, entsteht über den Stutzen 58 auch ein gewisser Saugeffekt in das innere der Hülse und überschüssige Flüssigkeit wird aus dem zu befeuchtenden Material entfernt. Auf dieser Weise lässt sich eine genaue Steuerung der Befeuchtung durchführen, wenn das Volumen des Behälters 52 und damit u.a. auch die genannte Saugwirkung an das zu befeuchtende Material 50 angepasst wird.

Auch dieses Ausführungsbeispiel gemäß Figur 3 kann dahingehend abgewandelt werden, das zwischen den elastischen Folien 44, 46 der Vakuumverpackung 40 ein Septum zum Einspritzen der Flüssigkeit angeordnet wird, insoweit ähnlich dem Ausführungsbeispiel gemäß den Figuren 1 und 2.

Wie eingangs erläutert ist, kann die Erfindung auch mit einem relativ starren Behälter realisiert werden, in den das poröse Implantat bzw. Transplantat evakuiert angeordnet wird.

## Patentansprüche

1. Verfahren zum Befeuchten eines im Wesentlichen biologischen medizinischen Implantates (10) oder Transplantates, bei dem das Implantat oder Transplantat vor dem Befeuchten evakuiert wird.

2. Vorrichtung zum Befeuchten eines im Wesentlichen biologischen medizinischen Implantates (10) oder Transplantates mit einer evakuierten Kammer (14), in der das Implantat oder das Transplantat enthalten ist.

3. Vorrichtung nach Anspruch 2, **dadurch gekennzeichnet, dass** Implantat oder Transplantat in einer elastischen Vakuumverpackung evakuiert enthalten ist.

4. Vorrichtung nach Anspruch 2 mit einem evakuierbaren Behälter.

5. Vorrichtung nach einem der Ansprüche 2, 3 oder 4, **dadurch gekennzeichnet, dass** eine erste Kammer (14) vorgesehen ist, in der das Implantat oder das Transplantat enthalten ist, und eine zweite Kammer (22), in der ein Befeuchtungsmittel enthalten ist.

6. Vorrichtung nach Anspruch 5, **dadurch gekennzeichnet, dass** die Kammern (14, 22) miteinander flüssigkeitsleitend verbindbar sind.

7. Vorrichtung nach einem der Ansprüche 2 bis 6, **dadurch gekennzeichnet, dass** die Vorrichtung ein Septum (18) aufweist zum Einspritzen einer Flüssigkeit.

8. Vorrichtung nach einem der Ansprüche 2 bis 7, **dadurch gekennzeichnet, dass** das Befeuchtungsmittel ein Medikament enthält.

9. Vorrichtung nach einem der Ansprüche 2 bis 8, **dadurch gekennzeichnet, dass** das Implantat oder das Transplantat aus Knochen besteht oder Knochenmaterial aufweist.

10. Vorrichtung nach einem der Ansprüche 2 bis 8, **dadurch gekennzeichnet, dass** das Implantat biologischen Ursprungs ist, insbesondere ein biologisches Binde- und Stützgewebe aufweist.

11. Vorrichtung nach einem der Ansprüche 2 bis 10, **dadurch gekennzeichnet, dass** das Implantat oder das Transplantat aus einem feuchtigkeitsaufnehmenden Material besteht, insbesondere ein poröser Körper (10) ist.

12. Verfahren zum Herstellen eines porösen medizinischen Implantates oder Transplantates, bei dem Knochen zu Partikeln zerkleinert wird, **dadurch gekennzeichnet, dass** die Partikel zu einem porösen Körper kompaktiert werden.

13. Verfahren nach Anspruch 12, **dadurch gekennzeichnet, dass** die Partikel Durchmesser bis 3 mm haben.

14. Verfahren nach einem der Ansprüche 12 oder 13, **dadurch gekennzeichnet, dass** die Partikel zumindest großteils langgestreckte Chip-Form haben.

15. Verfahren zum Herstellen eines porösen medizinischen Implantates oder Transplantates, bei dem ein gemäß einem der Ansprüche 12 bis 14 hergestellter Körper gemäß Anspruch 1 befeuchtet wird.
